(19) Europäisches Patentamt
European Patent Office
Office européen des brevets

(11) Veröffentlichungsnummer: **0 157 734**
**A1**

(12) # EUROPÄISCHE PATENTANMELDUNG


(21) Anmeldenummer: **85810097.7**

(22) Anmeldetag: **06.03.85**

(51) Int. Cl.[4]: **C 07 D 241/08**, C 07 D 241/38, C 08 K 5/34, C 09 D 3/80, C 09 D 7/12, C 08 L 33/08

(30) Priorität: **12.03.84 US 588462**

(43) Veröffentlichungstag der Anmeldung: **09.10.85 Patentblatt 85/41**

(84) Benannte Vertragsstaaten: **DE FR GB IT**



(71) Anmelder: **CIBA-GEIGY AG, Postfach, CH-4002 Basel (CH)**

(72) Erfinder: **Conetta, Thomas E., Walnut Hill Road, Bethel Connecticut 06801 (US)**
Erfinder: **Malherbe, Roger François, Dr., Bleichestrasse 7, CH-4058 Basle (CH)**
Erfinder: **Winter, Roland A.E., 23 Banksville Road, Armonk New York 10504 (US)**


(54) **Substituierte Piperazinone als Lichtschutzmittel.**


(57) Verbindungen der Formel I

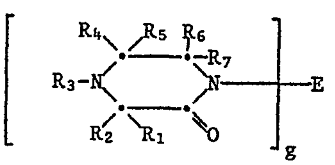

(I)

worin g 1 oder 2 ist, $R_3$ ein Acylrest, $R_1$, $R_2$, $R_4$ und $R_5$ Kohlenwasserstoffreste, $R_6$ und $R_7$ Wasserstoff oder Kohlenwasserstoffreste und E ein ein- oder zweiwertiger Substituent sind, sind wirkungsvolle Lichtschutzmittel für organische Polymere, insbesondere für Einbrennlacke auf Acrylharzbasis.


**EP 0 157 734 A1**

0157734

CIBA-GEIGY AG
Basel (Schweiz)

3-14792/=/CGC 1074

# Substituierte Piperazinone als Lichtschutzmittel

Die vorliegende Erfindung betrifft Verbindungen, die eine substituierte 4-Acyl-2-piperazinon-Gruppe enthalten und als Licht-und Hitzestabilisatoren für organische Materialien, insbesondere organische Polymere brauchbar sind, sowie die mit solchen Verbindungen stabilisierten Zusammensetzungen.

Sterisch gehinderte Amine mit einer 2,2,6,6-tetrasubstituierten Piperidingruppe sind seit längerem als wirkungsvolle Lichtschutzmittel für organische Materialien bekannt und haben als solche erhebliche wirtschaftliche Bedeutung erlangt. Solche gehinderten Amine sind z.B. von H.J. Heller und H.R. Blattmann in Pure & Applied Chemistry 36, 141-161 (1973) näher beschrieben.

Substituierte 2-Piperazinone als UV-Stabilisatoren für lichtempfindliche Kunststoffe sind beschrieben in den US-PS 4 190 571, 4 292 240 und 4 298 737. Die darin beschriebenen Verbindungen enthalten alle stark basische sekundäre oder tertiäre Aminogruppen, was die Verbindungen für bestimmte Verwendungszwecke ausschliesst. Im US 4 190 571 sind auch N-Carbalkoxyverbindungen (Urethane) nebenbei erwähnt, es wird dort jedoch nicht gesagt, wie solche Verbindungen hergestellt werden können und welche Eigenschaften sie haben.

Acylierte gehinderte Amine vom Piperazintyp sind beschrieben in den US-PS 4 046 737 und 4 066 615.

 

Es wurden nunmehr Verbindungen gefunden, die einen 4-Acyl-2-piperazinon-Rest enthalten und die sich in verbesserter Weise für die Stabilisierung von organischen Polymeren eignen.

Gegenstand der Erfindung sind Verbindungen der Formel I

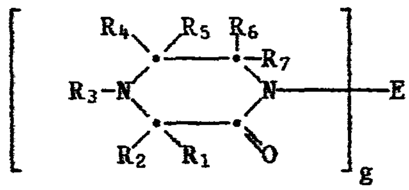

(I)

worin $R_1$ und $R_2$ unabhängig voneinander $C_1$-$C_{12}$-Alkyl, $C_5$-$C_{12}$-Cycloalkyl, $C_2$-$C_{14}$-Alkenyl oder $C_7$-$C_{15}$-Aralkyl oder $R_1$ und $R_2$ zusammen $C_4$-$C_6$-Alkylen bedeuten,
$R_3$ $C_1$-$C_{12}$-Alkanoyl, $C_3$-$C_8$-Alkenoyl oder Benzoyl bedeutet,
$R_4$ und $R_5$ unabhängig voneinander eine der für $R_1$ und $R_2$ gegebenen Bedeutungen haben,
$R_6$ und $R_7$ unabhängig voneinander eine der für $R_1$ und $R_2$ gegebenen Bedeutungen haben oder Wasserstoff sind,
g 1 oder 2 ist,
und E, wenn g 1 ist, Wasserstoff, $C_1$-$C_{24}$-Alkyl, $C_2$-$C_{14}$-Alkenyl, $C_7$-$C_{15}$-Aralkyl oder -Alkylaralkyl, $C_1$-$C_{12}$-Hydroxyalkyl, $C_2$-$C_{24}$-Alkanoyloxyalkyl, $C_4$-$C_{21}$-Alkenoyloxyalkyl oder $C_8$-$C_{18}$-Benzoyloxyalkyl bedeutet,
und wenn g 2 ist, unverzweigtes oder verzweigtes $C_2$-$C_{10}$-Alkylen bedeutet.

$R_6$ und $R_7$ sind vorzugsweise Wasserstoff.

$R_1$, $R_2$, $R_4$, $R_5$, $R_6$ und $R_7$ als Alkyl, Cycloalkyl, Alkenyl, Aralkyl oder Alkylaralkyl können z.B. folgende Bedeutungen haben:
Methyl, Ethyl, Isopropyl, sec-Butyl, n-Amyl, 2-Ethylhexyl, n-Decyl, n-Dodecyl; Vinyl, Allyl, Butenyl, Crotyl, Octenyl, Dodecenyl; Cyclopentyl, Cyclohexyl, Cyclooctyl, Cyclododecyl; Benzyl, 4-Methylbenzyl, 4-Butylbenzyl, 4-Octylbenzyl oder 2-Phenylethyl.

Vorzugsweise sind $R_1$, $R_2$, $R_4$ und $R_5$ unabhängig voneinander $C_1$-$C_4$-Alkyl, Allyl, Cyclohexyl oder Benzyl,insbesondere Methyl.

$R_3$ kann Alkanoyl sein, wie z.B. Formyl, Acetyl, Propionyl, Butyroyl, Valeroyl, Caproyl, Capryloyl, Decanoyl oder Lauroyl. $R_3$ kann Alkenoyl sein, wie z.B. Acryloyl, Methacryloyl, Hexenoyl oder Octenoyl. Vorzugsweise ist $R_3$ $C_2$-$C_8$-Alkanoyl, $C_3$-$C_4$-Alkenoyl oder Benzoyl, insbesondere Acetyl, Propionyl, Acryloyl oder Benzoyl.

$R_1$ und $R_2$, $R_4$ und $R_5$ sowie $R_6$ und $R_7$ können zusammen $C_4$-$C_6$-Alkylen sein, wie z.B. Tetra-, Penta- oder Hexamethylen.

E als Alkyl kann z.B. Methyl, Ethyl, Isopropyl, n-Amyl, 2-Ethylhexyl, n-Decyl, n-Dodecyl, n-Hexadecyl, n-Octadecyl, Eisocyl oder Tetracosyl sein. E als Alkenyl oder Aralkyl kann z.B. Vinyl, Allyl, Butenyl, Octyl, Octenyl oder Dodecenyl sein. E als Aralkyl oder Alkylaralkyl kann z.B. Benzyl, 4-Methylbenzyl, 4-Butylbenzyl, 4-Octylbenzyl oder 2-Phenylethyl sein.

Wenn E Hydroxyalkyl, Alkanoyloxyalkyl, Alkenoyloxyalkyl oder Benzoyloxyalkyl ist, so kann es z.B. Hydroxymethyl, 2-Hydroxyethyl, 2-Hydroxypropyl, 3-Hydroxypropyl, 2-Hydroxybutyl, 2-Hydroxydodecyl; Formyloxymethyl, Acetoxymethyl, 2-Acetoxyethyl, 2-Butyroyloxyethyl, 2-Octanoyloxyethyl, 2-Dodecanoyloxyethyl, 3-Eicosanoyloxypropyl; Acryloyloxymethyl, 2-Acryloyloxyethyl, 2-Methacryloyloxyethyl, 2-Oleyloxyethyl; Benzoyloxymethyl, 2-Benzoyloxyethyl oder 3-Benzoyloxypropyl sein.

Wenn $g = 1$ ist, ist E vorzugsweise $C_1$-$C_{12}$-Alkyl, Allyl, Benzyl, $C_2$-$C_3$-Hydroxyalkyl, $C_4$-$C_{14}$-Alkanoyloxyalkyl, $C_5$-$C_7$-Alkenoyloxyalkyl oder $C_9$-$C_{11}$-Benzoyloxyalkyl.

E als Alkylen kann z.B. 1,2-Ethylen, 1,2-Propylen, Tri-, Tetra-, Penta-, Hexa-, Octa- oder Dodecamethylen sein. Wenn $g = 2$ ist, ist E vorzugsweise Ethylen.

Die Verbindungen der Formel I können durch N-Acylierung der entsprechenden unsubstituierten Piperazinone der Formel II

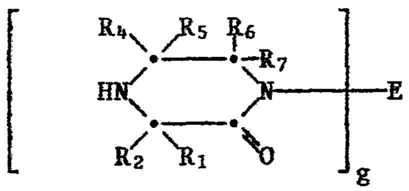

(II)

hergestellt werden. Dies kann nach den bekannten Methoden der N-Acylierung von sekundären Aminen geschehen, beispielsweise durch Umsetzung mit Carbonsäureanhydriden der Formel $(R_3)_2O$ oder Carbonsäurehalogeniden $R_3$-Hal, worin Hal Halogen, insbesondere Chlor oder Brom bedeutet. Ungesättigte Carbonsäurereste $R_3$ können auch durch Abspaltung von Halogenwasserstoff aus β-Halogencarbonsäureresten erhalten werden.

Die Zwischenprodukte der Formel II können nach den in der US-PS 4 190 571 angegebenen Methoden hergestellt werden, beispielsweise durch Reaktion von Cyanhydrinen mit Polyaminen.

Die Verbindungen der Formel I sind wirksame Stabilisatoren für organische Polymere. Beispiele hierfür sind:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS, MBS, ASA oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere wie Vinylchlorid-Vinylidenchlorid, Vinylchlorid-Vinylacetat oder Vinylidenchlorid-Vinylacetat.

8. Polymere, die sich von α,ß-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat-, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere wie z.B. Ethylenoxid enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylen-isophthalamid, sowie deren Block-Copolymere mit Polyethern wie z.B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten, oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren wie z.B. PP/EPDM, Polyamid 6/EPDM oder ABS, PVC/EVA, PVC/ABS, PVC/MBS, PC/ABS, PBTP/ABS.

Von besonderer Bedeutung ist die Verwendung der Verbindungen der Formel I in heissvernetzbaren Acryl- oder Alkydharzen, die häufig als Bindemittel für Einbrennlacke verwendet werden. Die Verbindungen der Formel I werden den Polymeren in einer Konzentration von 0,05 bis 5 Gew.-%, bezogen auf das zu stabilisierende Polymere zugesetzt, insbesondere in einer Konzentratrion von 0,1 bis 2,5 Gew.-%. Die Stabilisatoren können auch als Masterbatch zugesetzt werden, welcher die Stabilisatoren in einer Konzentration von etwa 2,5 bis 25 Gew.-% enthalten kann.

Die Stabilisatoren der Formel I können in einfacher Weise den Polymeren zugesetzt werden nach den hierfür gebräuchlichen Methoden. Dies kann in irgendeiner Stufe der Herstellung oder formgebenden Verarbeitung des Polymeren geschehen, beispielsweise durch Mischen in Pulverform oder in gelöster oder suspendierter Form.

Ausser den Verbindungen der Formel I können den Polymeren auch noch andere Stabilisatoren oder sonstige Additive zugesetzt werden. Beispiele hierfür sind die folgenden Stabilisatoren:

1. Antioxidantien

1.1. Alkylierte Monophenole

2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole

2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)

2,2'-Methylen-bis-[6-(α-methylbenzyl)-4-nonylphenol]
2,2'-Methlyen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercapto-butan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

<u>1.5 Benzylverbindungen</u>

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

<u>1.6. Acylaminophenole</u>

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

<u>1.7. Ester der ß-(3,5-Di-tert.butyl-4-hydroxyphenyl)-propionsäure</u> mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.8. Ester der ß-(5-tert.butyl-4-hydroxy-3-methylphenyl)-propionsäure mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.9. Amide der ß-(3,5-Di-tert.butyl-4-hydroxyphenyl)-propionsäure, wie z.B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

2. UV-Absorber und Lichtschutzmittel

2.1. 2-(2'-Hydroxyphenyl)-benztriazole, wie z.B. das 5'-Methyl-, 3',5'-Di-tert.butyl-, 5'-Tert.butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-ß,ß-diphenylacrylsäure-ethylester bzw. -isooctylester, α-Carbomethoxy-zimtsäuremethylester, α-Cyano-ß-methyl-p-methoxy-zimtsäuremethylester bzw. -butylester, α-Carbomethoxy-p-methoxy-zimtsäure-methylester, N-(ß-Carbomethoxy-ß-cyanovinyl)-2-methyl-indolin,

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethylpiperidyl)-sebacat
Bis-(1,2,2,6,6-pentamethylpiperidyl)-sebacat
n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin,
Tris-(2,2,6,6-tetramethyl-4-piperidyl)-nitrilotriacetat,
Tetrakis-(2,2,6,6-tetramethyl-4-piperidyl)-1,2,3,4-butantetracarbonsäure,
1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho-und para-Methoxy- sowie von o- und p-Ethoxy-di-substituierte Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit.

5. Peroxidzerstörende Verbindungen, wie z.B. Ester der ß-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(ß-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze

höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

Andere Additive, die zugesetzt werden können, sind z.B. Weichmacher, Gleitmittel, Füllstoffe, Glasfasern, Pigmente, optische Aufheller, Flammschutzmittel oder Antistatica.

Die folgenden Beispiele illustrieren die Erfindung im Detail, sollen jedoch keine Begrenzung der Erfindung darstellen. Die Temperaturangaban darin beziehen sich auf Grad Celsius.

Beispiel 1:
1,1'-Ethylen-bis(4-acetyl-3,3,5,5-tetramethyl-2-piperazinon)

31,5 g (0,1 Mol) 1,1'-Ethylen-bis(3,3,5,5-tetramethyl-2-piperazinon) - hergestellt gemäss Beispiel 6E in US-PS 4 190 571 aus N,N'-Bis(2-amino-2-methylpropyl)-ethylendiamin und Acetoncyanhydrin - werden in 102 g (1.0 Mol) Essigsäureanhydrid gelöst. Nach Zusatz von 3 Tropfen konzentrierter Schwefelsäure wird die Lösung 4 Stunden auf 130° erwärmt und dann auf Raumtemperatur ausgekühlt. Nach Zugabe von 200 ml Methylenchlorid wird das Reaktionsgemisch erst mit Wasser, dann mit $NaHCO_3$-Lösung gewaschen. Die Lösung wird über $MgSO_4$ getrocknet und im Vakuum eingedampft. Der Rückstand wird aus Toluol umkristallisiert und man erhält 30,4 g des oben benannten Produktes das bei 180-183° schmilzt (und bei 170° sintert).

Analyse ($C_{22}H_{38}N_4O_4$) Ber.: C 62,53 H 9,06 N 13,26 %

Gef.: C 62,6 H 9,0 N 13,40 %

Die Struktur wird durch das NMR-Spektrum bestätigt.

Beispiel 2:

1,1'-Ethylen-bis(4-propionyl-3,3,5,5-tetramethyl-2-piperazinon)

Analog zu Beispiel 1 erhält man diese Verbindung, wenn man statt Essigsäureanhydrid die äquivalente Menge Propionsäureanhydrid verwendet. Das Produkt schmilzt nach Umkristallisation aus Heptan/Toluol-Gemisch bei 135-137°.

Analyse ($C_{24}H_{42}N_4O_4$) Ber.: C 64,0 H 9,4 N 12,4 %

Gef.: C 64,2 H 9,6 N 12,3 %

Beispiel 3:

1,1'-Ethylen-bis(4-acryloyl-3,3,5,5-tetramethyl-2-piperazinon) Eine Lösung von 25 g (0,074 Mol) 1,1'-Ethylen-bis(3,3,5,5-tetramethyl-2-piperazinon) - hergestellt wie in Beispiel 1 - und 31,4 g (0,31 Mol) Triethylamin in 200 ml 1,2-Dichlorethan wird auf 0° gekühlt. Unter Kühlung auf 0° werden innerhalb 30 Minuten 19,2 g (0,15 Mol) 2-Chlorpropionylchlorid zugegeben. Das Reaktionsgemisch wird zuerst 2 Stunden bei 0 bis 5° gerührt, dann zum Rückfluss erwärmt und 2 Stunden auf Rückfluss gehalten. Das gebildete Triethylaminhydrochlorid wird abfiltriert, das Filtrat wird zuerst mit 1 n Salzsäure, dann mit Wasser und schliesslich mit Salzlösung gewaschen ung über $MgSO_4$ getrocknet. Das Lösungsmittel wird abdestilliert und der Rückstand aus Heptan/Chloroform umkristallisiert. Das erhaltene Produkt schmilzt bei 149-150°.

Analyse ($C_{24}H_{38}N_4O_4$) Ber.: C 64,5 H 8,6 N 12,5 %

Gef.: C 64,7 H 8,6 N 12,4 %

Die Struktur wird durch das NMR-Spektrum der Verbindung bestätigt.

Beispiel 4:

Stabilisierung eines wärmehärtbaren Acrylharzes

Ein Einbrennlack auf Basis eines wärmehärtbaren Acrylharzes, wie er in Automobil-Lackierungen als Decklack verwendet wird, wird mit den in Tabelle 1 angegebenen Stabilisatoren versehen. Die stabilisierten Lackproben werden in einer Schichtdicke von 63 µm auf Metallbleche aufgespritzt, die vorher mit einem Primer grundiert waren.

Die Proben werden dann 30 Minuten bei 122°C (250°F) eingebrannt.

Nach 3-wöchiger Lagerung bei 23°C/50 % RLF werden die Proben einem Schnellbewitterungstest im QUV-Gerät unterworfen. Hierbei werden die Proben alternierend je 1 Stunde bei 70°C mit UV-Licht bestrahlt und 4 Stunden bei 50°C beregnet (ASTM G53/77). Der Test dauert insgesamt 730 Stunden.

Von den Proben wird vor und nach der Bewitterung der 20°-Glanz gemäss ASTM D-523 und die Abbildungsschärfe DI (Distinctness of Image) gemäss ASTM D-16 der Oberfläche gemessen. Die prozentuelle Erhaltung dieser Werte nach der Bewitterung ist in Tabelle 1 aufgeführt.

Tabelle 1

| Stabilisator | Prozentuelle Erhaltung von | |
|---|---|---|
| | 20°-Glanz | DI |
| keiner | 4 % | 11 % |
| 2 % Verbindung des Beispiels 2 | 4 % | 28 % |
| 2 % Verbindung des Beispiels 3 | 61 % | 54 % |

Beispiel 5:

Verwendung in einem sauer härtbaren Einbrennlack

Ein sauer härtbarer Einbrennlack auf Basis von 70 % eines wärmehärtbaren Acrylharzes und 30 % eines Melaminharzes wird mit 0,1 Gew.-% p-Toluolsulfonsäure als Härtungskatalysator und 2 Gew.-% der in Tabelle 2 aufgeführten Stabilisatoren versetzt. Als Vergleich wird ein handelsübliches Lichtschutzmittel auf Basis eines sterisch gehinderten Piperidinderivats verwendet. Die Lackproben werden auf Stahlbleche aufgetragen und 60 Minuten bei 121°C (250°F) eingebrannt. Nach einer Lagerung von 1 Woche bei Normaltemperatur wird die Härte des Lackfilmes nach der Methode Knoop (ASTM B-778) gemessen. Ausserdem wird das allgemeine Aussehen des Lackes visuell beurteilt.

Tabelle 2

| Stabilisator | Knoop-Härte Nr. | Aussehen visuell |
|---|---|---|
| keiner | 8 | klar |
| 2 % Verbindung des Beispiels 1 | 7 | klar |
| 2 % Verbindung des Beispiels 2 | 8 | klar |
| 2 % Verbindung des Beispiels 3 | 8 | klar |
| 2 % Vergleichsprobe*) | 1 | Trübung mit Niederschlag |

*) Di-(2,2,6,6-tetramethylpiperidin-4-yl)-sebacat

Die erfindungsgemässen Stabilisatoren sind im säurehaltigen Lack löslich und ergeben klare Anstriche von ausgezeichneter Härte. Die Vergleichsprobe ergibt Interaktionen mit dem Säurekatalysator, wodurch eine ungenügende Härtung des Bindemittels und die Bildung von Niederschlägen resultiert.

Patentansprüche

1. Verbindungen der Formel I

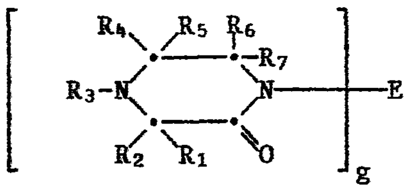

(I)

worin $R_1$ und $R_2$ unabhängig voneinander $C_1$-$C_{12}$-Alkyl, $C_5$-$C_{12}$-Cycloalkyl, $C_2$-$C_{14}$-Alkenyl oder $C_7$-$C_{15}$-Aralkyl oder $R_1$ und $R_2$ zusammen $C_4$-$C_6$-Alkylen bedeuten,
$R_3$ $C_1$-$C_{12}$-Alkanoyl, $C_3$-$C_8$-Alkenoyl oder Benzoyl bedeutet,
$R_4$ und $R_5$ unabhängig voneinander eine der für $R_1$ und $R_2$ gegebenen Bedeutungen haben,
$R_6$ und $R_7$ unabhängig voneinander eine der für $R_1$ und $R_2$ gegebenen Bedeutungen haben oder Wasserstoff sind,
g 1 oder 2 ist,
und E, wenn g 1 ist, Wasserstoff, $C_1$-$C_{24}$-Alkyl, $C_2$-$C_{14}$-Alkenyl, $C_7$-$C_{15}$-Aralkyl oder -Alkylaralkyl, $C_1$-$C_{12}$-Hydroxyalkyl, $C_2$-$C_{24}$-Alkanoyloxyalkyl, $C_4$-$C_{21}$-Alkenoyloxyalkyl oder $C_8$-$C_{18}$-Benzoyloxyalkyl bedeutet,
und wenn g 2 ist, unverzweigtes oder verzweigtes $C_2$-$C_{10}$-Alkylen bedeutet.

2. Verbindungen gemäss Anspruch 1, worin $R_1$, $R_2$, $R_4$ und $R_5$ unabhängig voneinander $C_1$-$C_4$-Alkyl, Allyl, Cyclohexyl oder Benzyl sind.

3. Verbindungen gemäss Anspruch 2, worin $R_1$, $R_2$, $R_4$ und $R_5$ Methyl sind.

4. Verbindungen gemäss Anspruch 1, worin $R_6$ und $R_7$ Wasserstoff sind.

5. Verbindungen gemäss Anspruch 1, worin $R_3$ $C_2$-$C_8$-Alkanoyl, $C_3$-$C_4$-Alkenoyl oder Benzoyl ist.

6. Verbindungen gemäss Anspruch 5, worin $R_3$ Acetyl, Propionyl, Acryloyl oder Benzoyl ist.

7. Verbindungen gemäss Anspruch 1, worin g 1 ist und E $C_1$-$C_{12}$-Alkyl, Allyl, Benzyl, $C_2$-$C_3$-Hydroxyalkyl, $C_4$-$C_{14}$-Alkanoyloxyalkyl, $C_5$-$C_7$-Alkenoyloxyalkyl oder $C_9$-$C_{11}$-Benzoyloxyalkyl ist.

8. Verbindungen gemäss Anspruch 1, worin g 2 und E Ethylen ist.

9. Die Verbindung 1,1'-Ethylen-bis(4-acetyl-3,3,5,5-tetramethyl-2-piperazinon) gemäss Anspruch 1.

10. Die Verbindun 1,1'-Ethylen-bis(4-propionyl-3,3,5,5-tetramethyl-2-piperidon) gemäss Anspruch 1.

11. Die Verbindung 1,1'-Ethylen-bis(4-acryloyl-3,3,5,5-tetramethyl-2-piperazinon).

12. Gegen Lichtabbau stabilisiertes organisches Polymer, enthaltend 0,05 bis 5 Gew.-% einer Verbindung des Anspruches 1.

13. Stabilisiertes Polymer gemäss Anspruch 12, das ein wärmehärtbares Acrylharz oder ein Einbrennlack auf Basis eines wärmehartbaren Acrylharzes ist.

14. Polymeres Material gemäss Anspruch 12, enthaltend als Stabilisator die Verbindung des Anspruches 11.

15. Verfahren zum Stabilisieren von organischen Polymeren, die von Licht abgebaut werden können, durch Zusatz von 0,05 bis 5 Gew.-% einer Verbindung des Anspruches 1.

16. Verfahren gemäss Anspruch 15 zum Stabilisieren eines wärmehärtbaren Acrylharzes oder Acrylharzlackes.

17. Verfahren gemäss Anspruch 15 durch Zusatz der Verbindung des Anspruches 11.

FO 7.3/SA/cw*

0157734

Europäisches Patentamt

# EUROPÄISCHER RECHERCHENBERICHT

Nummer der Anmeldung

EP 85 81 0097

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 001 284 (B.F. GOODRICH CO.) * Seite 5, Zeile 30 - Seite 9, Zeile 30; Seite 11, Zeile 12 - Seite 12, Zeile 15; Beispiel 6E * & US-A-4 190 571, US-A-4 292 240 (Kat. D) | 1-17 | C 07 D 241/08<br>C 07 D 241/38<br>C 08 K 5/34<br>C 09 D 3/80<br>C 09 D 7/12<br>C 08 L 33/08 |
| Y | EP-A-0 052 073 (CIBA GEIGY AG) * Seite 1, Zeile 1 - Seite 3, Zeile 24; Seite 13, Zeile 6 - Seite 15, Zeile 15 * | 1-17 | |
| A | DE-A-2 606 819 (HOECHST AG) * Ansprüche * | 1,12 | |
| A | DE-A-2 418 540 (SANKYO CO. LTD.) * Ansprüche * | 1,12 | **RECHERCHIERTE SACHGEBIETE (Int. Cl.4)** |
| A | DE-A-2 321 526 (CIBA GEIGY AG) * Ansprüche * | 1,12 | C 07 D 241/00<br>C 08 K 5/00<br>C 09 D 3/00<br>C 09 D 7/00<br>C 08 L 33/00 |

Der vorliegende Recherchenbericht wurde für alle Patentansprüche erstellt.

| Recherchenort | Abschlußdatum der Recherche | Prüfer |
|---|---|---|
| BERLIN | 11-06-1985 | VAN AMSTERDAM L.J.P. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument